# EUROPÄISCHE PATENTANMELDUNG

(11) **EP 2 332 475 A1**
(43) Veröffentlichungstag der Anmeldung: **15.06.2011**
(21) Anmeldenummer: 09015356.0
(22) Anmeldetag: 11.12.2009
(51) Int. Cl.: A61B 17/86, A61B 17/70, A61B 17/88

(54) **Knochenschraube**

(71) Anmelder: Böhm, Heinrich, Dr., 99425 Weimar (DE)
(72) Erfinder: Böhm, Heinrich, Dr., 99425 Weimar (DE); Burger, Andreas, 78532 Tuttlingen (DE); Wenzler, Klaus, 78665 Frittlingen (DE); Widmaier, Gerd, 78532 Tuttlingen (DE)
(74) Vertreter: Modrow, Stephanie

(57) **Zusammenfassung**

Die Erfindung betrifft eine Knochenschraube (10) mit einem kopfseitigen Ende (11) und einer Spitze (12), welche eine in Längsrichtung von dem kopfseitigen Ende (11) bis zu der Spitze (12) verlaufende Durchgangsöffnung (14) aufweist, wobei im Bereich des kopfseitigen Endes (11) der Durchgangsöffnung (14) erste Mittel zum Halten eines durch die Durchgangsöffnung (14) geführten Fadens (80) oder Drahts angeordnet sind.

## Beschreibung

Die Erfindung betrifft eine Knochenschraube gemäß dem Oberbegriff des Patentanspruchs 1.

Bekannt sind Knochenschrauben mit einem kopfseitigen Ende und einer Spitze, welche eine in Längsrichtung von dem kopfseitigen Ende bis zu der Spitze verlaufende Durchgangsöffnung aufweist.

Mit Hilfe derartiger Knochenschrauben werden beispielsweise Stabsysteme in Wirbelkörpern verankert. Die Durchgangsöffnung dient bekannterweise dazu, einen Führungsdraht zunächst in den Körper des Patienten bis zu dem Wirbelkörper einführen zu können, um anschließend die Knochenschraube über den Führungsdraht korrekt platzieren zu können.

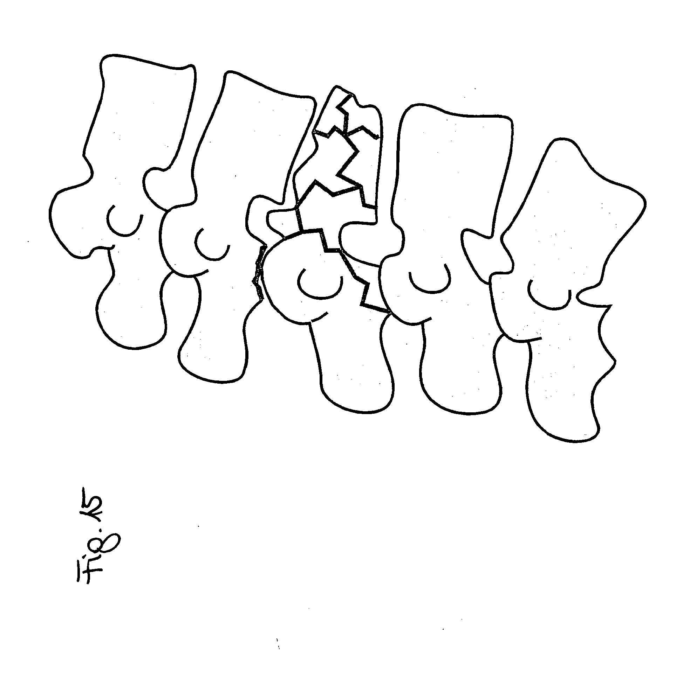

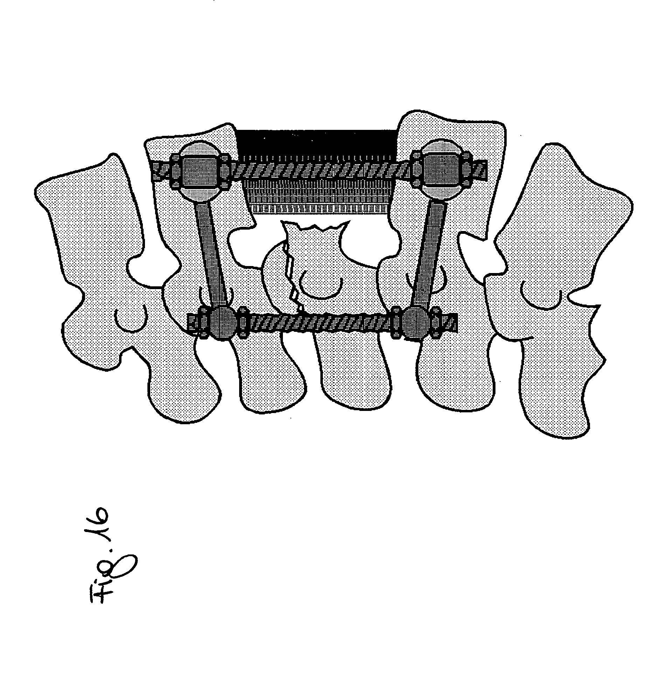

Derzeit sind folgende operative Maßnahmen zur Stabilisierung der Wirbelsäule bekannt. Einerseits erfolgen mechanische Stabilisierungen von dorsal mit Hilfe von Pedikelschrauben. Pedikelschrauben sind Knochenschrauben, die in die seitlich des Rückenmarkskanals verlaufende Wirbelanteile, die Pedikel, eingebracht und anschließend an den Schraubenköpfen miteinander verbunden werden. Die Pedikelschrauben werden dabei in offener Technik oder in minimalinvasiver Technik von einem rückenseitigen Zugang nach operativer Ablösung der Rückenmuskulatur in die Wirbelkörper eingeschraubt und anschließend durch Längsträger, beispielsweise Platten oder Stäbe, mechanisch fest miteinander verbunden. Dabei müssen bei einem nicht mehr tragfähigen Wirbelkörper, beispielsweise einem aufgrund von Tumor oder Unfall gebrochenen Wirbelkörper (vgl. Fig. 15), immer auch die beiden benachbarten Wirbelkörper mit fixiert werden, so dass mindestens drei Wirbelkörper und zwei Bandscheiben behelligt sind (vgl. Fig. 16). Damit soll der gebrochene oder auf sonstige Art und Weise beschädigte Wirbelkörper so lange mechanisch gegen die Schwerkraft gehalten werden, bis die knöcherne Fusion als biologische Lösung greift. Der gebrochene Wirbelkörper wird oft durch eine Wirbelkörperprothese zumindest teilweise ersetzt (vgl. Fig. 16).

In Fällen, in denen aus biologischen Gründen oder Krankheitsgründen keine knöcherne Heilung mehr erreichbar ist, sollte eine lebenslange Versorgung gewährleistet sein. Problem dabei ist insbesondere bei schlechter Knochenqualität, insbesondere auch bei Osteoporosen, dass die knöchernen Ankerpunkte, insbesondere dadurch, dass die Wirbelkörper weit von ihrer Schwerelinie entfernt gegen die Schwerkraft gehalten werden müssen, nachgeben, dass es zu einer sogenannten Anschlussdekompensation kommt und dass aufgrund des Umstands der erforderlich gewordenen operativen Versorgung das Problemfeld an der Wirbelsäule noch vergrößert wird und weitere Operationen erforderlich werden.

Eine alternative Maßnahme zur Stabilisierung beschädigter oder geschwächter Wirbelkörper ist die Vertebroplastie oder Kyphoplastie. Bei diesen Verfahren wir in minimalinvasiver Technik von dorsal Zement über den Pedikel in den Wirbelkörper eingespritzt. Nachteilig bei diesen Verfahren ist, dass ein großer Fremdkörper implantiert wird, der im weiteren Verlauf beispielsweise einer Wirbelkörperfraktur gerade verhindert, dass die Frakturpartner wieder im Sinne einer biologischen Lösung zusammenwachsen. Daher ist diesen Verfahren für eine Reihe von Frakturen insbesondere bei jungen Menschen nicht anwendbar. Schließlich besteht die Möglichkeit, gebrochene Wirbelkörper von vorn zu rekonstruieren. Dies erfordert nach herkömmlicher Technik einen großen, durch den Brustkorb geführten transthrokalen Eingriff oder auch einen minimalinvasiven endoskopisch assistierten Eingriff, in der Regel mit Entfernung des Wirbelkörpers und Rekonstruktion unter Verwendung von metallischen Implantaten. Aufgrund der Anatomie kann bei einer Thorakotomie oder Thorakophlenolumbotomie jeweils nur auf der Zugangsseite die Wirbelsäule von seitlich, beispielsweise mit Platten- oder Stabsystemen vorne fixiert werden. Häufig reicht dies jedoch nicht aus, um eine belastungsstabile Versorgung zu gewährleisten.

Die Aufgabe der Erfindung besteht darin, eine bekannte Knochenschraube weiterzuentwickeln, um insbesondere zusätzliche und verbesserte Behandlungsmethoden des Patienten zu erlauben.

Die Aufgabe der Erfindung wird gelöst durch eine Knochenschraube mit den Merkmalen des Patentanspruchs 1.

Vorteilhafte Ausgestaltungen und Weiterbildungen der Erfindung sind in den Unteransprüchen angegeben.

Die erfindungsgemäße Knochenschraube mit einem kopfseitigen Ende und einer Spitze, welche eine in Längsrichtung von dem kopfseitigen Ende bis zu der Spitze verlaufende Durchgangsöffnung aufweist, ist erfindungsgemäß derart ausgebildet, dass im Bereich des kopfseitigen Endes der Durchgangsöffnung erste Mittel zum Halten eines durch die Durchgangsöffnung geführten Fadens oder Drahts oder dergleichen angeordnet sind. Falls im Folgenden lediglich auf einen Faden Bezug genommen wird, ist immer auch ein Draht, eine Kordel, ein Metallkabel oder dergleichen als Alternative denkbar. Auf Grund der Tatsache, dass es mit der derart weitergebildeten Knochenschraube möglich ist, einen Faden in der Knochenschraube zu fixieren, wird beispielsweise eine neue Behandlungsmethode von gebrochenen Wirbelkörpern derart ermöglicht, dass durch zwei in dem Wirbel befestigte Knochenschrauben ein Faden derart gezogen wird, dass er die beiden gebrochenen Wirbelkörperteile miteinander verbindet und durch Halten des Fadens in den beiden Knochenschrauben eine Fixierung des gebrochenen Wirbelkörpers, insbesondere in Form einer Cerclage, ermöglicht wird. Weiterhin wird es ermöglicht, mehrere Wirbelkörper miteinander zu verbinden im Rahmen von Versteifungsoperationen, oder auch zur Vermeidung von Versteifungsoperationen mehrere Wirbel dynamisch zu fixieren.

Besonders bevorzugt sind die ersten Mittel als Spannzange ausgebildet, um eine möglichst einfache Befestigungsmöglichkeit zu ermöglichen.

Gemäß einer bevorzugten Ausführungsform der Erfindung sind die ersten Mittel in die Durchgangsöffnung der Knochenschraube einsetzbar, wobei sie den Faden klemmend halten. Auf diese Weise wird eine besonders benutzerfreundliche Knochenschraube bereitgestellt.

Die ersten Mittel können beispielsweise klemmend oder selbsthemmend in die Durchgangsöffnung der Knochenschrauben eingesetzt werden. Gemäß einer besonders bevorzugten Ausführungsform der Erfindung weisen die ersten Mittel jedoch ein Außengewinde auf, mit welchem sie in ein Innengewinde im Bereich des kopfseitigen Endes der Durchgangsöffnung der Knochenschraube einsetzbar sind, um auf diese Weise eine eindeutige Positionierung der ersten Mittel relativ zu der Knochenschraube bereitzustellen.

Gemäß einer bevorzugten Ausführungsform der Erfindung weist die Durchgangsöffnung der Knochenschraube in einem Abschnitt einen Konus auf, welcher bei Einsetzen der ersten Mittel in die Knochenschraube Klemmbacken der ersten Mittel derart zusammendrückt, dass der durch eine Durchgangsöffnung der ersten Mittel verlaufende Faden klemmend gehalten wird. Auf diese Weise erfolgt eine besonders einfache Fixierung des Fadens.

Gemäß einer besonders bevorzugten Ausführungsform der Erfindung wird eine Vorrichtung zum Befestigen eines Fadens in der erfindungsgemäßen Knochenschraube bereitgestellt.

Vorzugsweise weist die Vorrichtung zweite Mittel zum Halten des Fadens oder Drahts, dritte Mittel zum Spannen des Fadens oder Drahts und vierte Mittel zum Fixieren der ersten Mittel der Knochenschraube in der Knochenschraube bei bespanntem Faden oder Draht auf. Für viele Behandlungsmethode genügt es nicht, den Faden lediglich in der Knochenschraube zu fixieren, sondern es ist wesentlich, dass der Faden vor dem Fixieren gespannt wird, insbesondere um beispielsweise bei einem Wirbelbruch die Wirbelkörperteile zuverlässig aneinander zu fixieren. Daher ist es zunächst notwendig, einen durch die Knochenschraube geführten Faden zu halten, anschließend zu spannen und erst nach dem Spannen in der Knochenschraube in gespanntem Zustand zu fixieren.

Vorzugsweise weist die Vorrichtung ein Führungsrohr auf, durch welches der Faden oder Draht führbar ist und an welchem die zweiten Mittel, die dritten Mittel und die vierten Mittel angeordnet sind. Auf diese Weise kann mit Hilfe einer einzigen Vorrichtung der Faden an der Knochenschraube in gespanntem Zustand fixiert werden.

Besonders bevorzugt sind die zweiten Mittel als Halte-Spannzange ausgebildet, um eine möglichst einfache Befestigungsmöglichkeit zu ermöglichen.

Gemäß einer bevorzugten Ausführungsform sind die dritten Mittel als Verstellschraube ausgebildet, da somit auf einfache Art und Weise ein stufenloses Spannen des Fadens ermöglicht wird.

Gemäß einer besonders bevorzugten Ausführungsform der Erfindung sind die zweiten Mittel in eine Ausnehmung der dritten Mittel einsetzbar, wobei sie den Faden oder Draht klemmend halten. Auf diese Weise wird eine besonders benutzerfreundliche Vorrichtung bereitgestellt.

Gemäß einer bevorzugten Weiterbildung der Erfindung weisen die zweiten Mittel ein Außengewinde auf, mit welchem sie in ein in der Ausnehmung angeordnetes Innengewinde der dritten Mittel einsetzbar sind, um auf diese Weise eine eindeutige Positionierung der zweiten Mittel relativ zu den dritten Mitteln bereitzustellen.

Vorzugsweise weist die Ausnehmung der dritten Mittel in einem Abschnitt einen Konus auf, welcher bei Einsetzen der zweiten Mittel in die dritten Mittel Klemmbacken der zweiten Mittel derart zusammendrückt, dass der durch eine Durchgangsöffnung der zweiten Mittel verlaufende Faden oder Draht klemmend gehalten wird. Auf diese Weise erfolgt eine besonders einfache Fixierung des Fadens.

Gemäß einer bevorzugten Ausführungsform der Erfindung sind die dritten Mittel zum Spannen des Fadens oder Drahts, insbesondere einschließlich der zweiten Mittel, relativ zu dem Führungsrohr der Vorrichtung verdrehbar. Auf diese Weise ergibt sich eine einfach zu handhabende und kompakt aufgebaute Vorrichtung.

Aus dem gleichen Grund sind vorzugsweise die vierten Mittel als Hohlrohr ausgebildet, durch welche der Faden oder Draht führbar ist.

Eine besonders bevorzugte Ausführungsform der Erfindung sieht vor, dass die dritten Mittel und die ersten Mittel gegensinnige Drehrichtung aufweisen. Dadurch wird der Faden beim Spannen mit Hilfe der dritten Mittel in eine Richtung verdreht, beim Klemmen mit Hilfe der ersten Mittel jedoch in entgegengesetzte Richtung verdreht, so dass eine Beschädigung des Fadens durch axial gleichsinnige Rotation vermieden wird.

Gemäß einer besonders bevorzugten Weiterbildung der Erfindung sind die vierten Mittel axial verschiebbar und um die Längsachse verdrehbar in dem Führungsrohr der Vorrichtung gelagert. Auf diese Weise wird eine Handhabung der vierten Mittel nach Art eines Schraubendrehers in einfacher und kompakter Weise ermöglicht.

Die Erfindung wird anhand der nachfolgenden Figuren ausführlich erläutert. Es zeigt
- Fig. 1:: eine perspektivische Ansicht einer Knochenschraube und einer Vorrichtung zum Fixieren eines Fadens in der Knochenschraube gemäß der Erfindung mit einem vierten Mittel in einer ersten Position,
- Fig. 2:: einen Längsschnitt durch Fig. 1,
- Fig. 3:: eine Ausschnittsvergrößerung aus Fig. 2,
- Fig. 4:: eine weitere perspektivische Ansicht gemäß Fig. 1 mit dem vierten Mittel in einer zweiten Position,
- Fig. 5:: einen Längsschnitt durch Fig. 4,
- Fig. 6:: eine Ausschnittsvergrößerung aus Fig. 5,
- Fig. 7:: die Knochenschraube gemäß Fig. 1 mit durchgeführtem Faden und auf den Faden aufgefädelter Spannzange,
- Fig. 8:: eine Ausschnittsvergrößerung aus Fig. 7,
- Fig. 9:: die Vorrichtung zum Befestigen des Fadens in der Knochenschraube in auf die Knochenschraube gemäß Fig. 7 aufgesetztem Zustand,
- Fig. 10:: eine Ausschnittsvergrößerung aus Fig. 9,
- Fig. 11:: die Knochenschraube und die Vorrichtung gemäß Fig. 9 mit Verstellschraube und Halte-Spannzange,
- Fig. 12:: eine Ausschnittsvergrößerung aus Fig. 11,
- Fig. 13:: die Vorrichtung gemäß Fig. 11 mit Verstellschraube und Haltespannzange in einer geänderten Position und
- Fig. 14:: eine Ausschnittsvergrößerung aus Fig. 13.

In den Figuren sind verschiedene Ansichten einer Knochenschraube 10 und einer Vorrichtung 40 zum Befestigen eines Fadens 80 in der Knochenschraube 10 dargestellt. Gleiche Bezugsziffern bezeichnen gleiche Teile, wobei zur besseren Übersicht nicht sämtliche Bezugsziffern in sämtlichen Figuren dargestellt sind.

Die Knochenschraube 10 weist ein kopfseitiges Ende 11 und eine Spitze 12 auf, wobei das kopfseitige Ende 11 insbesondere als kugelförmiger Kopf 13 ausgebildet ist. An den Kopf 13 schließt sich ein Schaft mit einem Außengewinde 16 der Knochenschraube 10 an. Vom kopfseitigen Ende 11 bis zur Spitze 12 der Knochenschraube 10 verläuft in Längsrichtung der Knochenschraube 10 eine Durchgangsöffnung 14, wobei der Durchmesser der Durchgangsöffnung 14 im Bereich des kopfseitigen Endes 11 größer ist als im Bereich der Spitze 12. Insbesondere verjüngt sich der Durchmesser im Übergangsbereich zwischen dem Kopf 13 und dem das Außengewinde 16 tragenden Schaft nach Art eines Konus 19. Im Bereich des kopfseitigen Endes 11 der Knochenschraube 10, insbesondere im Bereich des Kopfes 13, ist ein Innengewinde 18 in der Durchgangsöffnung 14 der Knochenschraube 10 angeordnet. Im Bereich des kopfseitigen Endes 11 ist zudem in Längsrichtung der Knochenschraube 10 eine unrunde Ausnehmung 15 angeordnet, in welche ein entsprechendes Werkzeug zum Eindrehen der Knochenschraube 10 in einen Knochen eingesetzt werden kann. Das Innengewinde 18 ist in Längsrichtung an die Ausnehmung 15 angeordnet.

Durch die Durchgangsöffnung 14 der Knochenschraube 10 kann ein Faden 80 geführt werden. Statt des Fadens 80 kann auch ein Draht, eine Kordel, ein Metallkabel oder Ähnliches verwendet werden. Dabei kann es sich einerseits um ein Führungselement, beispielsweise einen Führungsdraht, handeln, das zunächst in den Patienten eingeführt wird, um anschließend entlang dieses Fadens 80 die Knochenschraube 10 korrekt platzieren zu können. Andererseits kann der Faden 80 jedoch beispielsweise dazu verwendet werden, gebrochene Wirbelkörper derart zu fixieren, dass der Faden 80 nach Art einer Schlaufe oder Cerclage durch die gebrochenen Wirbelkörper oder um benachbarte Wirbelkörper geführt wird und die beiden Enden des Fadens 80 in jeweils einer Knochenschraube 10 fixiert werden.

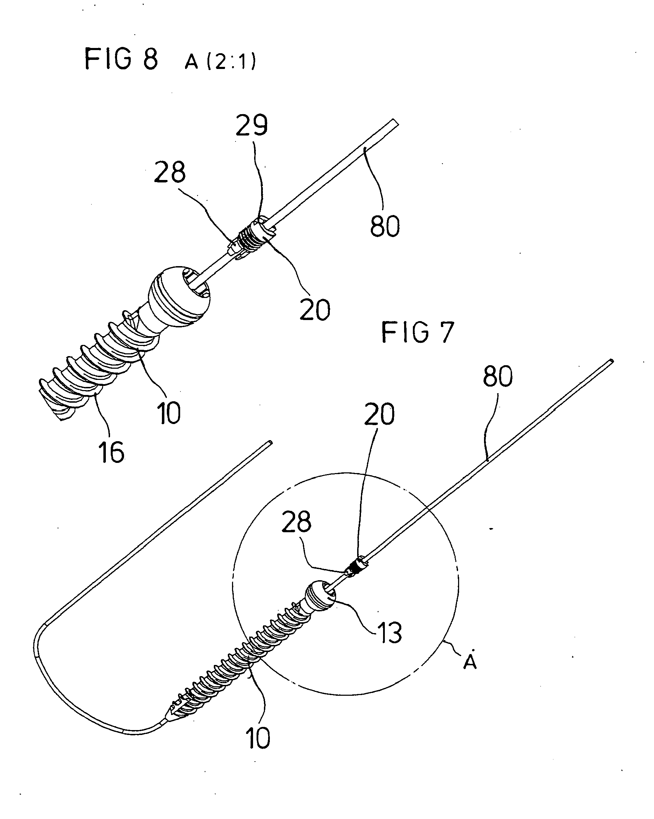

Zum Halten oder Fixieren des Fadens 80 in der Knochenschraube 10 ist ein erstes Mittel vorgesehen, welches insbesondere als Spannzange 20 ausgebildet ist. Die Spannzange 20 weist ein kopfseitiges Ende 21 und eine Spitze 22 auf, wobei in Längsrichtung von dem kopfseitigen Ende 21 bis zur Spitze 22 eine Durchgangsöffnung 24 angeordnet ist. Die Spannzange 20 weist dabei zumindest abschnittsweise ein Außengewinde 26 auf. Die Spannzange 20 kann in die Durchgangsöffnung 14 der Knochenschraube 10 eingesetzt werden. Dabei greift das Außengewinde 26 der Spannzange 20 in das Innengewinde 18 der Knochenschraube 10 ein. Im Bereich der Spitze 22 der Spannzange 20 sind Klemmbacken 28 angeordnet (vgl. insbesondere Fig. 7 und 8), welche, sobald die Spannzange 20 über das Außengewinde 26 in dass Innengewinde 18 der Knochenschraube 10 eingedreht wird, durch den Konus 19 der Knochenschraube 10 in Richtung auf die Längsachse zusammengedrückt werden können. Ist durch die Durchgangsöffnung 24 der Spannzange 20 ein Faden 80 geführt (vgl. Figuren 7 und 8), wird dieser durch die Klemmbacken 28 klemmend fixiert, sobald die Spannzange 20 weit genug in die Durchgangsöffnung 14 der Knochenschraube 10 eingedreht ist. Um die Spannzange 20 einfach in die Knochenschraube 10 eindrehen zu können, weist die Spannzange 20 in ihrer dem kopfseitigen Ende 21 zugewandten Stirnseite eine schlitzartige Ausnehmung 29 auf, in welche beispielsweise ein Schraubendreher einsetzbar ist.

Um den Faden 80 nach der Durchführung durch die Knochenschraube 10 spannen und in gespanntem Zustand fixieren zu können, ist eine Vorrichtung 40 vorgesehen. Die Vorrichtung 40 weist ein Führungsrohr 42 mit einer Längsachse A, einem ersten Ende 42a und einem zweiten Ende 42b sowie einer von dem ersten Ende 42a zu dem zweiten Ende 42b reichenden Durchgangsöffnung 44 auf.

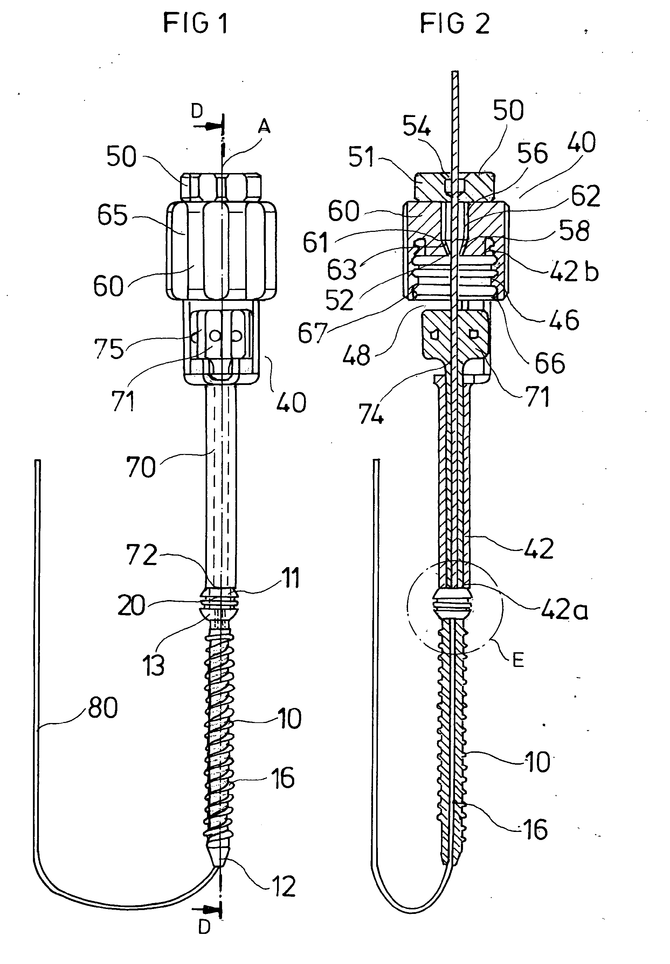

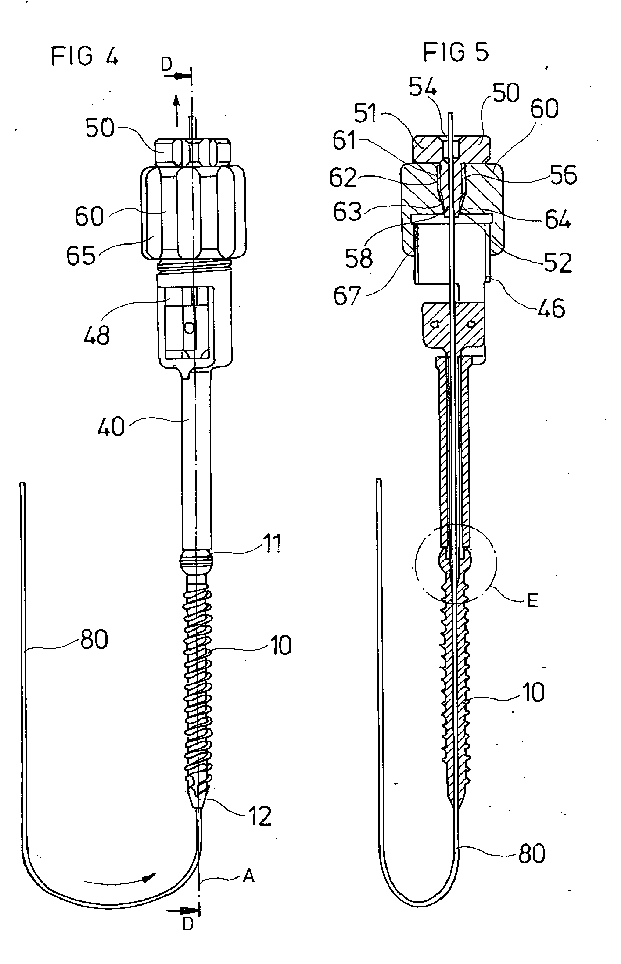

Zum Halten des Fadens 80 sind an der Vorrichtung 40 zweite Mittel ausgebildet, welche insbesondere als Halte-Spannzange 50 ausgebildet sind. Die Halte-Spannzange 50 weist ein kopfseitiges Ende 51 und eine Spitze 52 auf, wobei von dem kopfseitigen Ende 51 bis zur Spitze 52 eine Durchgangsöffnung 54 vorhanden ist. Die Halte-Spannzange 50 weist zumindest abschnittsweise ein Außengewinde 56 auf, wobei weiterhin im Bereich der Spitze 52 der Haltespannzange 50 Klemmbacken 58 vorgesehen sind (vgl. Figuren 2 und 5). Zwischen den Klemmbacken 58 kann bei entsprechendem Druck radial von außen ein durch die Durchgangsöffnung 54 geführter Faden 80 klemmend gehalten werden.

Zum Spannen des Fadens 80 sind an der Vorrichtung 40 weiterhin dritte Mittel vorgesehen, welche als Verstellschraube 60 ausgebildet sind. Die Verstellschraube 60 ist etwa zylindrisch mit einer Deckenfläche 60a und einer Bodenfläche 60b ausgebildet. Ausgehend von der Deckenfläche 60a ist in der Verstellschraube 60 eine Ausnehmung 61 in axialer Richtung angeordnet, in welcher ein erstes Innengewinde 62 angeordnet ist, das in einen Konus 63 mündet. Ausgehend von der Bodenfläche 60b ist in die Verstellschraube 60 eine weitere Ausnehmung 66 angeordnet, in welcher ein zweites Innengewinde 67 angeordnet ist. Die Ausnehmung 61 und die Ausnehmung 66 sind in axialer Richtung miteinander verbunden, so dass eine Durchgangsöffnung 64 in der Verstellschraube 60 gebildet wird. Die Ausnehmung 66 weist dabei einen größeren Innendurchmesser auf als die Ausnehmung 61.

In das erste Innengewinde 62 kann die Halte-Spannzange 50 eingesetzt werden derart, dass das Außengewinde 56 der Halte-Spannzange 50 in das erste Innengewinde 62 der Verstellschraube 60 eingreift. Wird die Halte-Spannzange 50 genügend weit in das erste Innengewinde 62 eingedreht, werden die Klemmbacken 58 durch den Konus 63 der Verstellschraube 60 derart zusammengedrückt, dass ein durch die Durchgangsöffnung 54 der Halte-Spannzange 50 und die Durchgangsöffnung 64 der Verstellschraube 60 geführter Faden 80 klemmend gehalten wird.

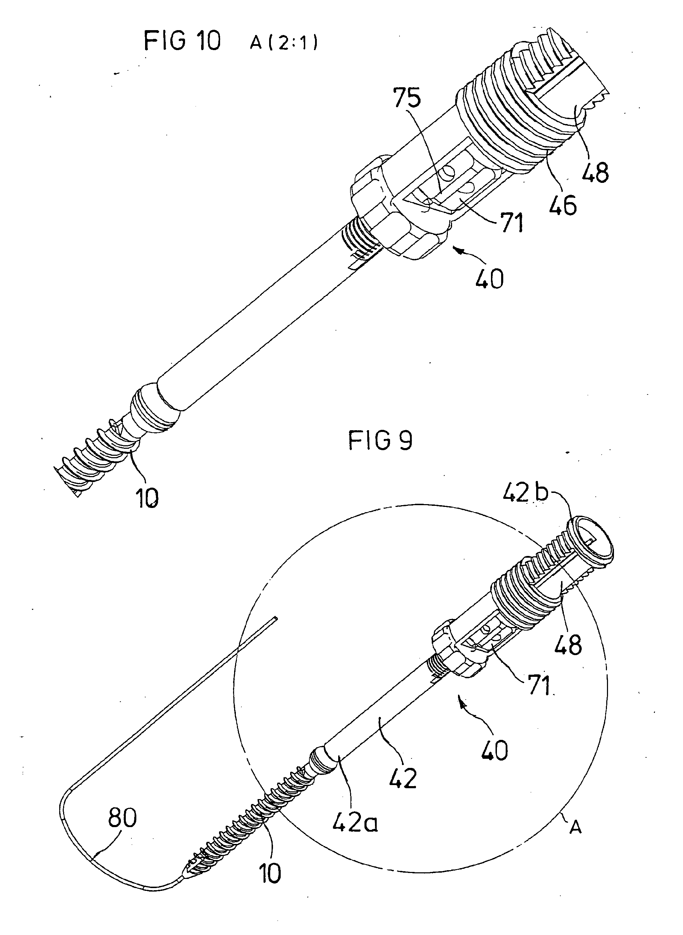

Über das zweite Innengewinde 67 kann die Verstellschraube 60 auf das Außengewinde 46 im Bereich des ersten Endes 42a des Führungsrohrs 42 der Vorrichtung 40 (vgl. insbesondere Figuren 9 und 10) aufgeschraubt werden. Das Schrauben wird für einen Benutzer durch eine auf der Außenfläche der Verstellschraube 60 angeordnete Riffelung 65 erleichtert.

Der durch die Knochenschraube 10 geführte Faden 80 kann anschließend durch das Führungsrohr 42, die Durchgangsöffnung 64 der Verstellschraube 60 und die Durchgangsöffnung 54 der Halte-Spannzange 50 geführt werden. Zum Halten des Fadens 80 wird zunächst die Halte-Spannzange 50 über das Außengewinde 56 in das erste Innengewinde 62 der Verstellschraube 60 soweit eingeschraubt, bis die Klemmbacken 58 der Halte-Spannzange 50 durch den Konus 63 der Verstellschraube 60 zusammengedrückt und der Faden 80 somit klemmend in der Halte-Spannzange 50 fixiert wird.

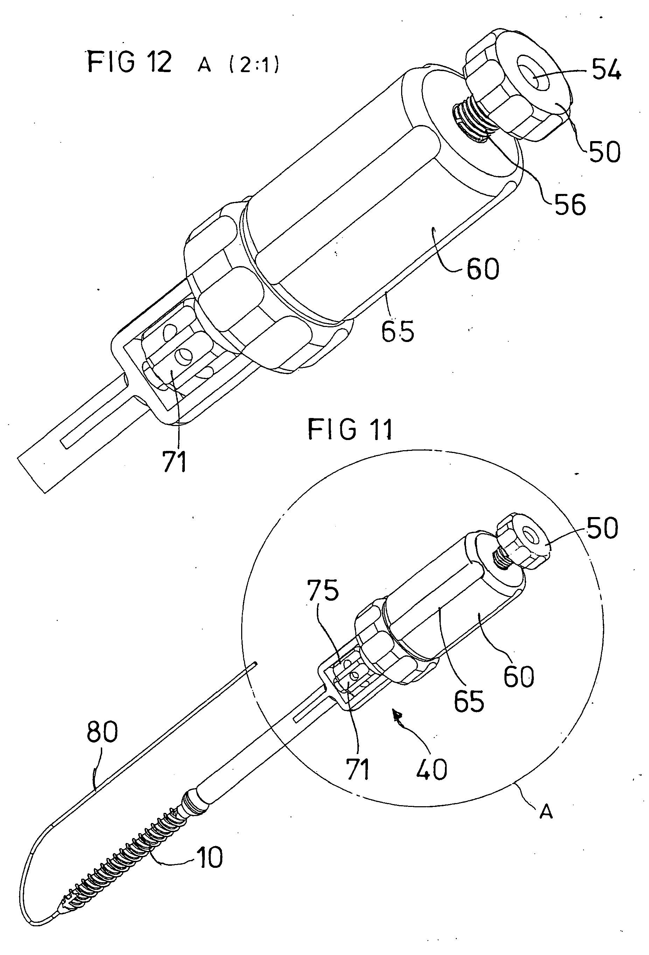

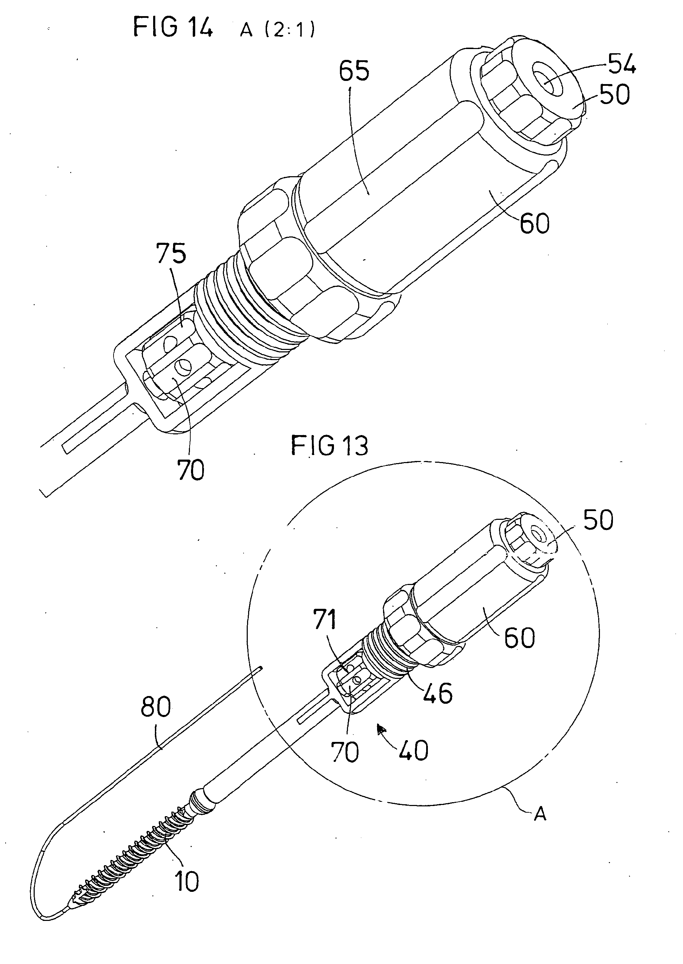

Zum Spannen des Fadens 80 wird das Führungsrohr 42 der Vorrichtung 40 mit seinem zweiten Ende 42b auf den Kopf 13 der Knochenschraube 10 aufgesetzt und die Verstellschraube 60 ist bereits bevor der Faden 80 mit der Halte-Spannzange 50 gegriffen wird soweit wie möglich in Richtung auf das zweite Ende 42b der Vorrichtung 40 aufgeschraubt (vgl. insbesondere Figuren 11 und 12). Nach Fixieren des Fadens 80 mit Hilfe der Haltespannzange 50 wird die Verstellschraube 60 in Richtung auf das erste Ende 42a der Vorrichtung 40 verdreht, wobei die Halte-Spannzange 50 einschließlich des fixierten Fadens 80 mitgenommen wird und der Faden 80 auf diese Weise gespannt wird (vgl. insbesondere Figuren 13 und 14).

Zum Fixieren des Fadens 80 in gespanntem Zustand in der Knochenschraube 10 muss anschließend die Spannzange 20 der Knochenschraube 10 festgedreht werden, damit der Faden 80 durch die Klemmbacken 28 der Spannzange 20 in der Knochenschraube 10 fixiert wird. Dabei weist vorzugsweise die Spannzange 20 eine gegensinnige Drehrichtung zu der Verstellschraube 60 auf, beispielsweise dadurch, dass entweder das Außengewinde 26 der Spannzange 20 oder das zweite Innengewinde 67 der Verstellschraube 60 als Linksgewinde ausgebildet ist, um eine Beschädigung des Fadens 80 durch zwei gleichsinnige Rotationen zu vermeiden.

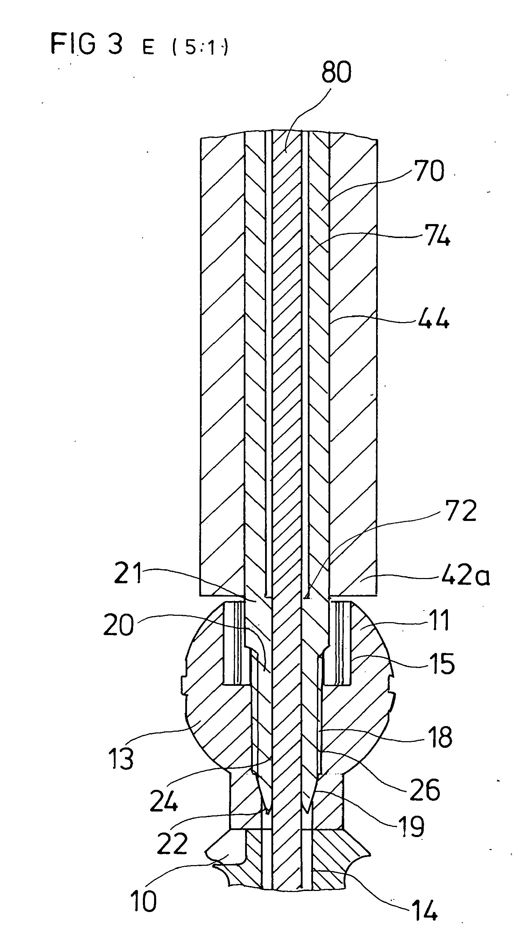

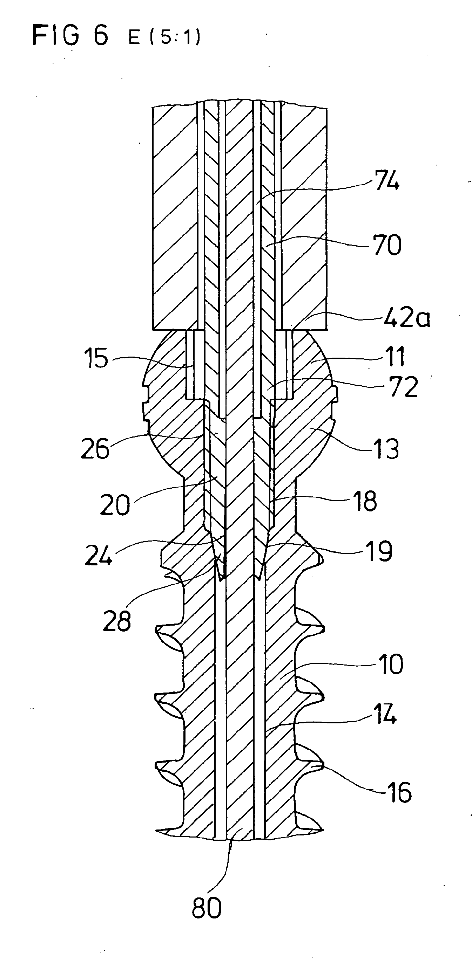

Dazu sind an der Vorrichtung 40 vierte Mittel zum Fixieren der ersten Mittel, d. h. der Spannzange 20, in der Knochenschraube 10 bei gespanntem Faden 80 vorgesehen. Die vierten Mittel sind als Hohlrohr 70 mit einem kopfseitigen Ende 71 und einem Arbeitsende 72 ausgebildet, wobei zwischen dem kopfseitigen Ende 71 und dem Arbeitsende 72 eine Durchgangsöffnung 74 angeordnet ist. Das Hohlrohr 70 ist insbesondere konzentrisch in das Führungsrohr 42 der Vorrichtung 40 eingesetzt. Weiterhin weist das Führungsrohr 42 der Vorrichtung 40 eine quer verlaufende radiale Öffnung 48 auf, durch welche das kopfseitige Ende 71 des Hohlrohrs 70 zugänglich ist. Das Hohlrohr 70 ist in dem Führungsrohr 42 axial verschiebbar und um seine Längsachse verdrehbar angeordnet. Die axiale Bewegung ist dabei dadurch beschränkt, dass das kopfseitige Ende 71 einen größeren Durchmesser aufweist als ein sich in Richtung auf das Arbeitsende 72 des Hohlrohrs 70 anschließender hohler Schaft, wobei das kopfseitige Ende 71 in einer entsprechenden Aufweitung des Führungsrohrs 42 im Bereich des ersten Endes 42a zu liegen kommt, an welches sich ein hohler Schaft geringeren Durchmessers anschließt. Das Arbeitsende 72 des Hohlrohrs 70 ist nach Art eines Schraubendrehers ausgebildet. Der Faden 80 wird bei Durchführung durch die Vorrichtung 40 durch die Durchgangsöffnung 74 des Hohlrohrs 70 gezogen. Nach Spannen des Fadens 80 mit Hilfe der Verstellschraube 60 kann die Spannzange 20 durch das Hohlrohr 70 in der Knochenschraube 10 fixiert werden, wozu insbesondere das Arbeitsende 72 des Hohlrohrs 70 in die schlitzartige Ausnehmung 29 der Spannzange 20 eingesetzt wird und das Hohlrohr 70 in der Vorrichtung 40 um seine Längsachse gedreht wird, um die Spannzange 20 mit dem Außengewinde 26 in das Innengewinde 18 der Knochenschraube 10 einzudrehen und die Klemmbacken 28 derart in dem Konus 19 zusammenzudrücken, dass der Faden 80 bei gespanntem Zustand in der Knochenschraube 10 fixiert wird. Die Figuren 1, 2 und 3 zeigen dabei das Hohlrohr 70 in einer auf die Spannzange 20 aufgesetzten Position, die Figuren 4, 5 und 6 zeigen das Hohlrohr 70 nach Eindrehen der Spannzange 20 in die Knochenschraube 10. Das Drehen des Hohlrohrs 70 wird für einen Benutzer dadurch vereinfacht, dass im Bereich des kopfseitigen Endes 71 des Hohlrohrs 70 auf der Außenseite eine Riffelung 75 angeordnet ist.

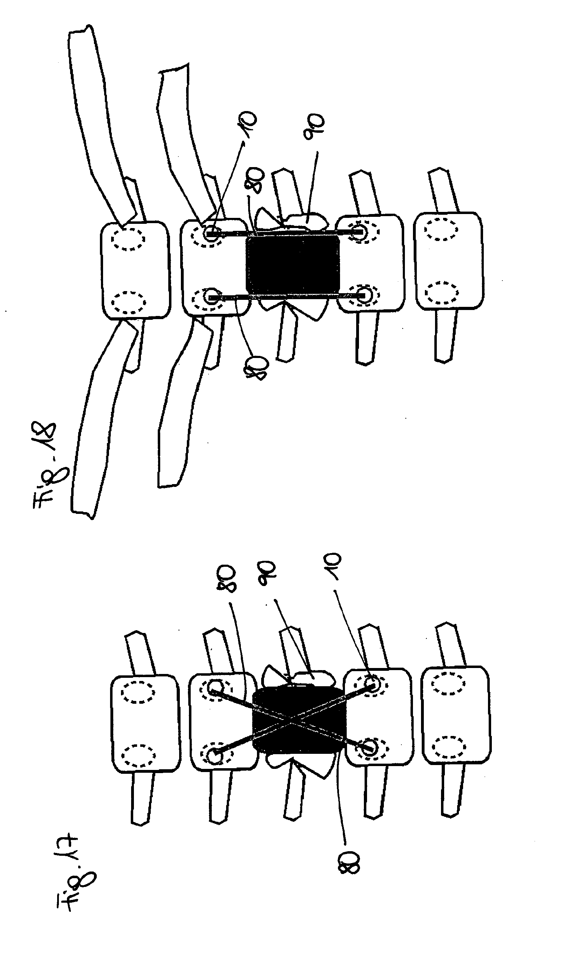

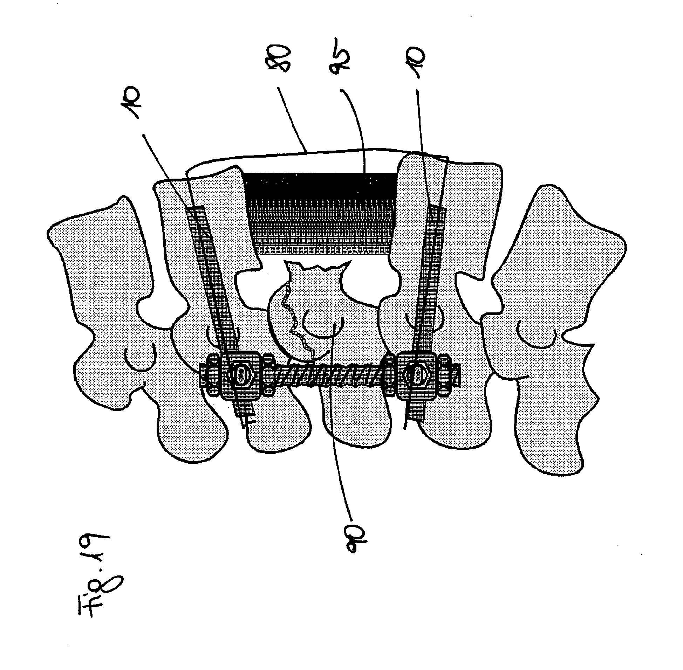

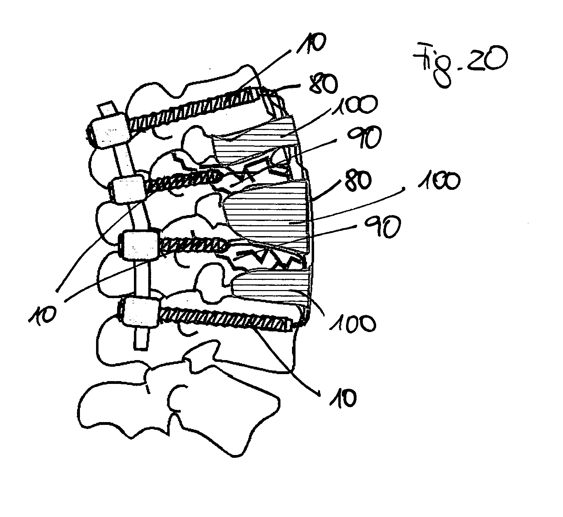

Durch die Knochenschraube 10 wird folgendes Operationsverfahren ermöglicht, welches anhand der Figuren 17 bis 20 erläutert wird. In den oder die geschwächten oder gebrochenen Wirbelkörper 90 werden entweder in offener Technik oder minimalinvasiv die Knochenschrauben 10 eingebracht. Über die Durchgangsöffnung 14 einer dieser Knochenschrauben 10 wird der Faden 80, beispielsweise eine Kordel aus reißfestem Fadenmaterial, durch gefädelt, der oder die rekonstruierte Wirbelkörper 90 wird vorne längs (vgl. Fig. 17) oder quer (vgl. Fig. 18 und 19) umfahren und über eine zweite dieser Knochenschrauben 10 wieder zurück geführt. Das Prinzip ist also eine Cerclage. Damit werden die Teile des mechanisch nicht mehr tragfähigen Wirbelkörpers 90 so stabil zusammengebunden, dass sie den Körper des Patienten wieder tragen können und die Fragmente des gebrochenen Wirbelkörpers 90 in guter Stellung zusammenheilen können. Gegebenenfalls ist es aber auch nötig, Teile des nicht mehr tragfähigen Wirbelkörpers 90 durch eine Wirbelkörperprothese 95 zu ersetzen (vgl. Fig. 19). Die Knochenschrauben 10 erlauben weiterhin in operativer Technik von vorne am Wirbelkörper 90 den Einsatz weiterer Zusatzimplantate 100, sogenannter Scaffolds, zur Stabilisierung und Anregung der Knochenneubildung (vgl. Fig. 20). Die Zusatzimplantate 100 sind insbesondere als textile Implantate, beispielsweise als auffaltbare netzartige Strukturen, ausgebildet und sind beispielsweise an die vorderen Wirbelsäulenabschnitte anatomisch anpassbar. Die Zusatzimplantate 100 werden insbesondere von dem Faden 80 wie eine Art Auffanggitter oder Außenwand eines Pakets gefasst. Nach Rückführung des Fadens 80 durch eine weitere Knochenschraube 80 kann der betroffene Wirbelsäulenabschnitt wie ein Paket verschnürt werden. Die Fixierung des Fadens 80 erfolgt dorsal. Damit kann im Gegensatz zu herkömmlichen Methoden und Implantaten die Wirbelsäule auch vorne von beiden Seiten symmetrisch stabilisiert werden. Anstelle der indirekten Technik kann durch eine Paketverschnürung eine direkte Stabilisierung erreicht werden. Um die Wirbelkörperfragmente mechanisch gleichwertig zu fixieren, sind wesentlich weniger starke Kräfte erforderlich. Die indirekte Krafteinleitung über gesunde Nachbar-Wirbelkörper wird vermieden, so dass der Eingriff wesentlich weniger belastend und komplikationsbehaftet ist. Das Verfahren ist auch bei mehreren betroffenen Wirbelkörpern zur Rekonstruktion anwendbar.

### Bezugszeichenliste

- 10: Knochenschraube
- 11: kopfseitiges Ende
- 12: Spitze
- 13: Kopf
- 14: Durchgangsöffnung
- 15: Ausnehmung
- 16: Außengewinde
- 18: Innengewinde
- 19: Konus

- 20: Spannzange (1. Mittel)
- 21: kopfseitiges Ende
- 22: Spitze
- 24: Durchgangsöffnung
- 26: Außengewinde
- 28: Klemmbacken
- 29: schlitzartige Aufnehmung

- 40: Vorrichtung
- 42: Führungsrohr
- 42a: erstes Ende
- 42b: zweites Ende
- 44: Durchgangsöffnung
- 46: Außengewinde
- 48: Öffnung

- 50: Halte-Spannzange (2. Mittel)
- 51: kopfseitiges Ende
- 52: Spitze
- 54: Durchgangsöffnung
- 56: Außengewinde
- 58: Klemmbacken

- 60: Verstellschraube (3.Mittel)
- 60a: Deckenfläche
- 60b: Bodenfläche
- 61: Ausnehmung
- 62: erstes Innengewinde
- 63: Konus
- 64: Durchgangsöffnung
- 65: Riffelung
- 66: Ausnehmung
- 67: zweites Innengewinde

- 70: Hohlrohr (4. Mittel)
- 71: kopfseitiges Ende
- 72: Arbeitsende
- 74: Durchgangsöffnung
- 75: Riffelung

- 80: Faden

- 90: Wirbelkörper
- 95: Wirbelkörperprothese

- 100: Zusatzimplantat

- A: Längsachse

## Patentansprüche

1. Knochenschraube (10) mit einem kopfseitigen Ende (11) und einer Spitze (12), welche eine in Längsrichtung von dem kopfseitigen Ende (11) bis zu der Spitze (12) verlaufende Durchgangsöffnung (14) aufweist,
**dadurch gekennzeichnet, dass** im Bereich des kopfseitigen Endes (11) der Durchgangsöffnung (14) erste Mittel zum Halten eines durch die Durchgangsöffnung (14) geführten Fadens (80) oder Drahts angeordnet sind.

2. Knochenschraube nach Anspruch 1,
**dadurch gekennzeichnet, dass** die ersten Mittel als Spannzange (20) ausgebildet sind.

3. Knochenschraube nach Anspruch 1 oder 2,
**dadurch gekennzeichnet, dass** die ersten Mittel in die Durchgangsöffnung (14) der Knochenschraube (10) einsetzbar sind und dabei den Faden (80) oder Draht klemmend halten.

4. Knochenschraube nach einem der vorhergehenden Ansprüche, **dadurch gekennzeichnet, dass** die ersten Mittel ein Außengewinde (26) aufweisen, mit welchem sie in ein Innengewinde (18) im Bereich des kopfseitigen Endes (11) der Durchgangsöffnung (14) einsetzbar sind.

5. Knochenschraube nach einem der vorhergehenden Ansprüche, **dadurch gekennzeichnet, dass** die Durchgangsöffnung (14) der Knochenschraube (10) in einem Abschnitt einen Konus (19) aufweist, welcher bei Einsetzen der ersten Mittel in die Knochenschraube (10) Klemmbacken (28) der ersten Mittel derart zusammendrückt, dass der durch eine Durchgangsöffnung (24) der ersten Mittel verlaufende Faden (80) oder Draht klemmend gehalten wird.

6. Vorrichtung (40) zum Befestigen eines Fadens (80) oder eines Drahts in einer Knochenschraube (10) nach einem der vorhergehenden Ansprüche.

7. Vorrichtung nach Anspruch 6,
**dadurch gekennzeichnet, dass** die Vorrichtung (40) zweite Mittel zum Halten des Fadens (80) oder Drahts, dritte Mittel zum Spannen des Fadens (80) oder Drahts und vierte Mittel zum Fixieren der ersten Mittel der Knochenschraube (10) in der Knochenschraube (10) bei gespanntem Faden (80) oder Draht aufweist.

8. Vorrichtung nach Anspruch 7,
**dadurch gekennzeichnet, dass** die Vorrichtung (40) ein Führungsrohr (42) aufweist, durch welches der Faden (80) oder Draht führbar ist und an welchem die zweiten Mittel, die dritten Mittel und die vierten Mittel angeordnet sind.

9. Vorrichtung nach einem der Ansprüche 7 bis 8,
**dadurch gekennzeichnet, dass** die zweiten Mittel als Halte-Spannzange (50) ausgebildet sind.

10. Vorrichtung nach einem der Ansprüche 7 bis 9,
**dadurch gekennzeichnet, dass** die dritten Mittel als Verstellschraube (60) ausgebildet sind.

11. Vorrichtung nach einem der Ansprüche 7 bis 10,
**dadurch gekennzeichnet, dass** die zweiten Mittel in eine Ausnehmung (61) der dritten Mittel einsetzbar sind und dabei den Faden (80) oder Draht klemmend halten.

12. Vorrichtung nach Anspruch 11,
**dadurch gekennzeichnet, dass** die zweiten Mittel ein Außengewinde (56) aufweisen, mit welchem sie in ein in der Ausnehmung (61) angeordnetes Innengewinde (62) der dritten Mittel einsetzbar sind.

13. Vorrichtung nach Anspruch 11 oder 12,
**dadurch gekennzeichnet, dass** die Ausnehmung (61) der dritten Mittel in einem Abschnitt einen Konus (63) aufweist, welcher bei Einsetzen der zweiten Mittel in die dritten Mittel Klemmbacken (58) der zweiten Mittel derart zusammendrückt, dass der durch eine Durchgangsöffnung (54) der zweiten Mittel verlaufende Faden (80) oder Draht klemmend gehalten wird.

14. Vorrichtung nach einem der Ansprüche 7 bis 13,
**dadurch gekennzeichnet, dass** die dritten Mittel zum Spannen des Fadens (80) oder Drahts, insbesondere einschließlich der zweiten Mittel, relativ zu dem Führungsrohr (42) der Vorrichtung (40) verdrehbar sind.

15. Vorrichtung nach einem der Ansprüche 7 bis 14,
**dadurch gekennzeichnet, dass** die dritten Mittel und die ersten Mittel gegensinnige Drehrichtung aufweisen.

16. Vorrichtung nach einem der Ansprüche 7 bis 15,
**dadurch gekennzeichnet, dass** die vierten Mittel als Hohlrohr (70) ausgebildet sind, durch welche der Faden (80) oder Draht führbar ist.

17. Vorrichtung nach einem der Ansprüche 7 bis 16,
**dadurch gekennzeichnet, dass** die vierten Mittel axial verschiebbar und um die Längsachse (A) verdrehbar in dem Führungsrohr (42) der Vorrichtung (40) gelagert sind.
